# EUROPEAN PATENT SPECIFICATION

(11) **EP 2 367 937 B1**
(45) Date of publication and mention of the grant of the patent: **17.04.2013**
(21) Application number: 09768434.4
(22) Date of filing: 25.11.2009
(51) Int. Cl.: C12N 15/00

(54) **METHOD FOR THE REDUCTION OF REPETITIVE SEQUENCES IN ADAPTER-LIGATED RESTRICTION FRAGMENTS**
VERFAHREN ZUR REDUZIERUNG VON WIEDERHOLUNGSSEQUENZEN IN ADAPTER-LIGIERTEN RESTRIKTIONSFRAGMENTEN
PROCÉDÉ POUR LA RÉDUCTION DE SÉQUENCES RÉPÉTITIVES DANS DES FRAGMENTS DE RESTRICTION LIGATURÉS À UN ADAPTATEUR

(30) Priority: 04.12.2008 US 119864 P
(43) Date of publication of application: 28.09.2011
(73) Proprietor: KEYGENE N.V., 6700 AE Wageningen (NL)
(72) Inventor: TREBBI, Daniele, 6700 AE WAGENINGEN (NL); VAN EIJK, Michael, Josephus, Theresia, NL-5373 EJ Herpen (NL)
(74) Representative: de Lang, Robbert-Jan
(86) International application number: PCT/NL2009/000232
(87) International publication number: WO 2010/064893

(56) References cited:
- EP-A1- 0 534 858
- WO-A1-01/06014
- WO-A1-2008/119084
- WO-A2-2008/070223
- MEUDT ET AL: "Almost Forgotten or Latest Practice? AFLP applications, analyses and advances" TRENDS IN PLANT SCIENCE, ELSEVIER SCIENCE, OXFORD, GB, vol. 12, no. 3, 16 March 2007 (2007-03-16) , pages 106-117, XP005920449 ISSN: 1360-1385
- PETERSON D G ET AL: "Efficient capture of unique sequences from eukaryotic genomes" TRENDS IN GENETICS, ELSEVIER SCIENCE PUBLISHERS B.V. AMSTERDAM, NL, vol. 18, no. 11, 1 November 2002 (2002-11-01), pages 547-550, XP004389596 ISSN: 0168-9525

## Description

The present invention relates to methods for reducing the amount repetitive sequences in genomes and to methods for enriching samples for low-copy sequences. The invention further relates to the use of renaturation kinetics based fractionation for the enrichment of adapter-ligated restriction fragments for low-copy sequences. The invention further relates to the use of double strand-specific nuclease (DSN) for the enrichment of adapter-ligated restriction fragments for low-copy sequences. The invention further relates to the use of samples enriched for low-copy fragments in fingerprint analysis, SNP mining and marker development.

### Background of the invention

Many genomes, in particular plants, contain repetitive sequences, i.e. parts, whether large or smaller, of the DNA that are repeated at several positions in a genome. In particular when genome sizes increase and in particular in plants, where genomes can have large sizes (e.g. Arabidopsis thaliana 130 Mb, Oryza sativa 430 Mb, Zea Mays 2400Mb, Barley 5300 Mb and Onion 15000 Mb per haploid genome). Some genomes such as pepper and maize are said to contain up to 80 % of repetitive sequences.

AFLP (EP534858, Vos et al 1995) is a successful technology for the development of markers and the identification of SNPs that are linked to a particular trait or QTL. To subsequently develop such AFLP markers or SNPs to PCR markers, i.e. markers that can be used with a normal PCR assay, the markers or SNPs have to be converted. The conversion is a laborious process that in many occasions is not successful (Brugmans et al. Nucleic Acids Research 2003 31 (10):e55) in particular due to the presence of repetitive sequences in the genome. Repetitive sequences do not yield useful markers or SNPs that can be linked to a trait or a quantitative trait locus. That in itself does not necessarily pose problem, but the presence of repetitive sequences hampers the subsequent conversion of AFLP markers to PCR markers. Therefore, there is a need to enrich restriction fragment mixtures obtained from DNA samples for low-copy sequences, because this will increase the probability for successful conversion to PCR markers and thereby improve the effectiveness of genetic marker development for traits

### Summary of the invention

The present inventors have now found that a combination of elements of the AFLP technology, DNA reassociation kinetics and DSN normalisation provides an efficient and reliable way of enriching a DNA sample for low copy sequences. The enrichment for low copy sequences is achieved, according to the invention by providing adapter-ligated restriction fragments from a DNA sample using a first restriction endonuclease, subjecting the adapter-ligated restriction fragments to renaturation kinetics based fractionation and subjecting the fractions to a DSN, restricting the remaining fragments with a second restriction endonuclease and ligating a second adapter to the restriction fragment to thereby obtain a low-copy enriched fraction of the starting DNA.

### Definitions:

In the following description and examples, a number of terms are used. In order to provide a clear and consistent understanding of the specification and claims, including the scope to be given such terms, the following definitions are provided. Unless otherwise defined herein, all technical and scientific terms used have the same meaning as commonly understood by one of ordinary skill in the art to which this invention belongs.

AFLP: AFLP refers to a method for selective amplification of nucleic acids based on digesting a nucleic acid with one or more restriction endonucleases to yield restriction fragments, ligating adaptors to the restriction fragments and amplifying the adaptor-ligated restriction fragments with at least one primer that is (part) complementary to the adaptor, (part) complementary to the remains of the restriction endonuclease, and that further contains at least one randomly selected nucleotide from amongst A,C, T, or G (or U as the case may be) at the 3'end of the primer. AFLP does not require any prior sequence information and can be performed on any starting DNA. In general, AFLP comprises the steps of:
(a) digesting a nucleic acid, in particular a DNA or cDNA, with one or more specific restriction endonucleases, to fragment the DNA into a corresponding series of restriction fragments;
(b) ligating the restriction fragments thus obtained with a double-stranded synthetic oligonucleotide adaptor, one end of which is compatible with one or both of the ends of the restriction fragments, to thereby produce adaptor-ligated, restriction fragments of the starting DNA;
(c) contacting the adaptor-ligated, restriction fragments under hybridizing conditions with one or more oligonucleotide primers that contain selective nucleotides at their 3'-end;
(d) amplifying the adaptor-ligated, restriction fragment hybridised with the primers by PCR or a similar technique so as to cause further elongation of the hybridised primers along the restriction fragments of the starting DNA to which the primers hybridised; and
(e) detecting, identifying or recovering the amplified or elongated DNA fragment thus obtained.

AFLP thus provides a reproducible subset of adaptor-ligated fragments. AFLP is described in EP 534858, US 6045994 and in Vos et al 1995. AFLP: a new technique for DNA fingerprinting. Nucleic Acids Research 23(21):4407-4414. Reference is made to these publications for further details regarding AFLP. The AFLP is commonly used as a complexity reduction technique and a DNA fingerprinting technology. Within the context of the use of AFLP as a fingerprinting technology, the concept of an AFLP marker has been developed.

AFLP marker: An AFLP marker is an amplified adaptor-ligated restriction fragment that is different between two samples that have been amplified using AFLP (fingerprinted), using the same set of primers. As such, the presence or absence of this amplified adaptor-ligated restriction fragment can be used as a marker that is linked to a trait or phenotype. With conventional slab gel electrophoresis , an AFLP marker shows up as a band in the gel with a certain mobility. Other electrophoretic techniques such as capillary electrophoresis may not refer to this as a band, but the concept remains the same, i.e. a nucleic acid with a certain length and mobility. Absence or presence of the band may be indicative of (or associated with) the presence or absence of the phenotype. AFLP markers typically involve polymorphisms in the restriction site of the endonuclease (type 1) or the in selective nucleotides (type 2). Occasionally, AFLP markers may involve indels (insertion-deletions in the restriction fragment (type 3).

Selective base or selective nucleotide: Located at the 3' end of the primer that contains a part that is complementary to the adaptor and a part that is complementary to the remains of the restriction site, the selective base is randomly selected from amongst A, C, T or G (or U as the case may be). By extending a primer with a selective base, the subsequent amplification will yield only a reproducible subset of the adaptor- ligated restriction fragments, i.e. only the fragments that can be amplified using the primer carrying the selective base. Selective nucleotides can be added to the 3'end of the primer in a number varying between 1 and 10.. Typically, 1-4 suffice. Both primers may contain a varying number of selective bases. With each added selective base, the subset reduces the amount of amplified adaptor-ligated restriction fragments in the subset by a factor of about 4. Typically, the number of selective bases used in AFLP is indicated by +N/+M, wherein one primer carries N selective nucleotides and the other primers carries M selective nucleotides. Thus, an EcoRI/MseI +1/+2 AFLP is shorthand for the digestion of the starting DNA with EcoRI and Msel, ligation of appropriate adaptors and amplification with one primer directed to the EcoRI restricted position carrying one selective base and the other primer directed to the Msel restricted site carrying 2 selective nucleotides. A primer used in AFLP that carries at least one selective nucleotide at its 3' end is also depicted as an AFLP-primer. Primers that do not carry a selective nucleotide at their 3' end and which in fact are complementary to the adaptor and the remains of the restriction site are sometimes indicated as AFLP+0 primers. The term selective nucleotide is also used for nucleotides of the target sequence that are located adjacent to the adaptor section and that have been identified by the use of selective primer as a consequence of which, the nucleotide has become known.

Sequencing: The term sequencing refers to determining the order of nucleotides (base sequences) in a nucleic acid sample, e.g. DNA or RNA. Many techniques are available such as Sanger sequencing and high-throughput sequencing technologies such as offered by 454 technologies (Roche Applied Science), Illumina Inc. and Applied BioSystems, Helicos and others..

Restriction endonuclease: a restriction endonuclease or restriction enzyme is an enzyme that recognizes a specific nucleotide sequence (target site) in a double-stranded DNA molecule, and will cleave both strands of the DNA molecule at or near every target site, leaving a blunt or a staggered end.

Frequent cutters and rare cutters: Restriction enzymes typically have recognition sequences that vary in number of nucleotides from 3, 4 (such as Msel) to 6 (EcoRI) and even 8 (NotI). The restriction enzymes used can be frequent and rare cutters. The term 'frequent' in this respect is typically used in relation to the term 'rare'. Frequent cutting endonucleases (aka frequent cutters) are restriction endonucleases that have a relatively short recognition sequence. Frequent cutters typically have 3-5 nucleotides that they recognise and subsequently cut. Thus, a frequent cutter on average cuts a DNA sequence every 64-512 nucleotides. Rare cutters are restriction endonucleases that have a relatively long recognition sequence. Rare cutters typically have 6 or more nucleotides that they recognise and subsequently cut. Thus, a rare 6-cutter on average cuts a DNA sequence every 1024 nucleotides, leading to longer fragments. It is observed again that the definition of frequent and rare is relative to each other, meaning that when a 4 bp restriction enzyme, such as Msel, is used in combination with a 5-cutter such as AvaII, AvaII is seen as the rare cutter and Msel as the frequent cutter.

Restriction fragments: the DNA molecules produced by digestion with a restriction endonuclease are referred to as restriction fragments. Any given genome (or nucleic acid, regardless of its origin) will be digested by a particular restriction endonuclease into a discrete set of restriction fragments. The DNA fragments that result from restriction endonuclease cleavage can be further used in a variety of techniques and can for instance be detected by gel electrophoresis.

Ligation: the enzymatic reaction catalyzed by a ligase enzyme in which two double-stranded DNA molecules are covalently joined together is referred to as ligation. In general, both DNA strands are covalently joined together, but it is also possible to prevent the ligation of one of the two strands through chemical or enzymatic modification of one of the ends of the strands. In that case the covalent joining will occur in only one of the two DNA strands.

Synthetic oligonucleotide: single-stranded DNA molecules having preferably from about 10 to about 50 bases, which can be synthesized chemically are referred to as synthetic oligonucleotides. In general, these synthetic DNA molecules are designed to have a unique or desired nucleotide sequence, although it is possible to synthesize families of molecules having related sequences and which have different nucleotide compositions at specific positions within the nucleotide sequence. The term synthetic oligonucleotide will be used to refer to DNA molecules having a designed or desired nucleotide sequence.

Adaptors: short double-stranded DNA molecules with a limited number of base pairs, e.g. about 10 to about 30 base pairs in length, which are designed such that they can be ligated to the ends of restriction fragments. Adaptors are generally composed of two synthetic oligonucleotides which have nucleotide sequences which are partially complementary to each other. When mixing the two synthetic oligonucleotides in solution under appropriate conditions, they will anneal to each other forming a double-stranded structure. After annealing, one end of the adaptor molecule is designed such that it is compatible with the end of a restriction fragment and can be ligated thereto; the other end of the adaptor can be designed so that it cannot be ligated, but this need not be the case (double ligated adaptors).

Adaptor-ligated restriction fragments: restriction fragments that have been capped by adaptors.

Primers: in general, the term primers refer to synthetic DNA molecules which can prime the synthesis of DNA. DNA polymerase cannot synthesize DNA de *novo* without a primer: it can only extend an existing DNA strand in a reaction in which the complementary strand is used as a template to direct the order of nucleotides to be assembled. We will refer to the synthetic oligonucleotide molecules which are used in a polymerase chain reaction (PCR) as primers.

DNA amplification: the term DNA amplification will be typically used to denote the *in vitro* multiplication of DNA molecules, using PCR yielding double stranded DNA or other other amplification methods such as GenomiPhi / RepliG yielding single stranded DNA. It is noted that yet other amplification methods exist and they may be used in the present invention without departing from the gist.

Repetitive sequence: a repetitive sequence is a sequence that is repeated many times, but at least two or more times in a DNA or a genome. Highly-repetitive sequence: a highly-repetitive sequence is a sequence that is repeated typically hundreds to thousands of times in a DNA or a genome. Examples are Tandem repeats such as satellite DNA, minisatellites and microsatellite and Interspersed repeats such as SINEs (Short INterspersed Elements) and LINEs (Long INterspersed Elements), (retro)transposons.

Low-copy sequence, low copy-number sequence, unique sequence: sequence that occurs only in a limited number of copies (or only in one copy) in a genome and can be used as a marker linked to a phenotype. Low-copy fraction: fraction of a DNA sample that is reduced in repetitive sequences and hence is enriched in low-copy sequences, i.e. a fraction from which repetitive sequences have been removed.

DSN (double strand-specific nuclease): a nuclease that specifically cuts double stranded DNA sequences (Shagin et al 2002 Genome Research 12 1935-1945, Zhulidov et al 2004 NAR 32 3 e37).

Renaturation kinetics based fractionation: a technique wherein low-copy sequences are separated from high-copy or repetitive sequences in a DNA sample . The procedure involves heating a sample of genomic DNA until it denatures into the single stranded-form, and then slowly cooling it, so the strands can pair back together. While the sample is cooling, measurements can be taken of how much of the DNA is base paired at each temperature.

The amount of single and double-stranded DNA can be measured by rapidly diluting the sample, which slows reassociation, and then binding the DNA to a hydroxylapatite column. The column can be eluted to elute the single-stranded DNA first and followed by elution of the double stranded DNA. The amount of DNA in these two solutions can be measured using a spectrophotometer. Since a sequence of single-stranded DNA needs to find its complementary strand to reform a double helix, common sequences renature more rapidly than rare sequences. Indeed, the rate at which a sequence will reassociate is proportional to the number of copies of that sequence in the DNA sample. A sample with a highly-repetitive sequence will renature rapidly, while complex sequences will renature slowly.

However, instead of simply measuring the percentage of double-stranded DNA versus time, the amount of renaturation is usually measured relative to a C₀t value. The C₀t value is the product of C₀ (the initial concentration of DNA), t (time in seconds), and a constant that depends on the concentration of cations in the buffer. Repetitive DNA will renature at low C₀t values, while complex and unique DNA sequences will renature at high C₀t values.

C₀t filtration: C₀t filtration is a technique that uses the principles of DNA renaturation kinetics to separate the repetitive DNA sequences that dominate many eukaryotic genomes from "gene-rich" single/low-copy sequences. This allows DNA sequencing to concentrate on the parts of the genome that are most informative and interesting, which will speed up the discovery of new genes and make the process more efficient (Peterson DG, Wessler SR, Paterson AH (2002). "Efficient capture of unique sequences from eukaryotic genomes". Trends Genet. 18 (11): 547-50, LLamoureux D, Peterson DG, Li W, Fellers JP, Gill BS (2005). "The efficacy of C0t-based gene enrichment in wheat (Triticum aestivum L.)". Genome 48 (6):1120-6, Yuan Y, SanMiguel PJ, Bennetzen JL (2003). "High-C0t sequence analysis of the maize genome". Plant J. 34 (2): 249-55.

### Detailed description of the invention

Thus, in a first embodiment the invention relates to a method for the reduction of repetitive sequences (enhancing the (relative) amount of low-copy sequences) in adapter-ligated restriction fragments comprising the steps of:
a. restricting a starting DNA in a sample with a first restriction endonuclease and ligating a first adapter to the restriction fragments to obtain adapter-ligated restriction fragments;
b. performing a renaturation kinetics based fractionation on the adapter-ligated restriction fragments obtained in step (a);
c. subjecting the renatured fractions of step (b) to a duplex specific nuclease (DSN);
d. restricting the restriction fragments obtained from step (c) with a second restriction endonuclease and ligating a second adapter to the restriction fragments;
e. obtaining a low-copy enriched fraction of the starting DNA.

The method according to the invention combines certain elements of AFLP (creation of adapter- ligated restriction fragments using a restriction enzyme and ligation of adapters) with certain elements of renaturation kinetics based fracturation (allowing high copy sequences to renature to become double stranded but low copy sequences to remain single-stranded), removing the double stranded sequences by DSN and, again AFLP (further creation of adapter-ligated restriction fragments using a second restriction enzyme. The results is that a fraction of the starting DNA is obtained that is enriched for the presence of low-copy sequences and reduced in the number of highly repetitive sequences.

In step (a) of the method according to the invention, a starting DNA is treated with a restriction endonuclease. The starting DNA can be any DNA such as genomic DNA, cDNA, BAC DNA, mitochondrial DNA, chloroplast DNA or mixtures of plant genomic DNA containing mitochondrial and chloroplast DNA. The first restriction endonuclease digests the starting DNA to yield restriction fragments. The first restriction endonuclease is preferably a rare cutter, such as a hexacutter, such as EcoRI, EcoRII, BamHI, Hindlll, PstI leading to restriction fragments which typically range in size between 1-4 kb in case an AT-rich endonuclease sich as EcoRI (recognition sequence GAATTC) is applied in and AT-rich organism such as most plant species. To these restriction fragments, first adapters are ligated to yield adapter-ligated restriction fragments.

In step (b) of the method according to the invention, the adapter-ligated restriction fragments, are subjected to a renaturation kinetics-based fractionation. Thus, the adapter-ligated restriction fragments are heated until denaturation into the single stranded-form, and then slowly cooled down, so the strands can pair back together. As the adapter-ligated restriction fragments are typically within the same length range due to the use of the resection endonuclease, the repetitive sequences will re-anneal within a determinable amount of time while the low-copy sequences will remain single stranded for a longer period of time. After cooling has proceeded long enough, the re-annealing is stopped, for instance by forced cooling. In renaturation kinetics-based fractionation disclosed in the art, the amount of single and double-stranded DNA is usually measured by rapidly diluting the sample, which slows reassociation, and then binding the DNA to a hydroxylapatite column after which the single stranded and double stranded fractions can be eluted.

In a preferred embodiment of the invention, the use of the column and the intermediate separation into fractions that essentially contain single stranded DNA or double stranded DNA, respectively, can be avoided by (directly) treating the sample with a double stranded nuclease in step (c) of the method. The double stranded nuclease specifically cuts double stranded DNA sequences (Shagin et al 2002 Genome Research 12 1935-1945, Zhulidov et al 2004 NAR 32 3 e37). As most of the repetitive sequences are now in double stranded form, the sample is reduced for adapter-ligated restriction fragments that are derived from repetitive sequences (or enriched for adapter-ligated restriction fragments that are derived from low-copy or unique sequences).

The renatured fractions of step (b), whether or not DSN treated in optional step (c), are subjected in step (d) of the method of the invention to a second digestion with a restriction endonuclease, followed by ligation of the adapter corresponding to the endonuclease. The resulting adapter-ligated restriction fragments, that, in a preferred embodiment, on each side of the restriction fragment carry a different adapter. Thus the result of the method of the current invention is that a set of adapter-ligated restriction fragments is obtained that is enriched for low-copy DNA (or reduced significantly in relative occurrence of repetitive DNA).

The obtained adapter-ligated restriction fragments can be used in subsequent fingerprinting technologies such as AFLP, for SNP mining etc.. Thus in one embodiment, the obtained low-copy DNA sample can be selectively amplified and fingerprinted to obtain higher quality fingerprints.

The Cₒt methodology is in itself well known for the normalisation of cDNA libraries, also in combination with DSN. Typically, C₀t-fragments are obtained by shearing or otherwise by fragmenting DNA in a more or less random manner. Prior to Cₒt analysis, fragments are typically size selected to reduce the effect that larger fragments renature quicker than shorter fragments, which could be detrimental to the desired result. By now performing a first endonuclease restriction and adapter ligation before Cₒt treatment and further fragmentation, the size of the fragments are more manageable and generally lie within a more limited size range. On the other hand, performing endonuclease restriction using two enzymes (and hence two adapters), would lead to a too large spread of lengths (in the area of 900-50 bp). By executing the method with a first restriction step, then a Cₒt treatment and a subsequent restriction step, the Cₒt treatment is performed on a set of adapter-ligated restriction fragments that have renaturation kinetics that are more homogeneous than those of fragment mixtures containing a wide range of large and small adapter-ligated fragments.

The method of the present invention is particularity useful to increase the success rate of conversion of AFLP markers (or other markers) to, for instance PCR markers and in the discovery of useful SNPs which can be turned into assays applicable on whole genomic DNA.

### Description of the Figures

Figure 1 Pepper results for Pepper E33/M49 and Pepper P14/M60 are
Figure 2 Maize results for Maize E35/M48 and Maize P12/M50

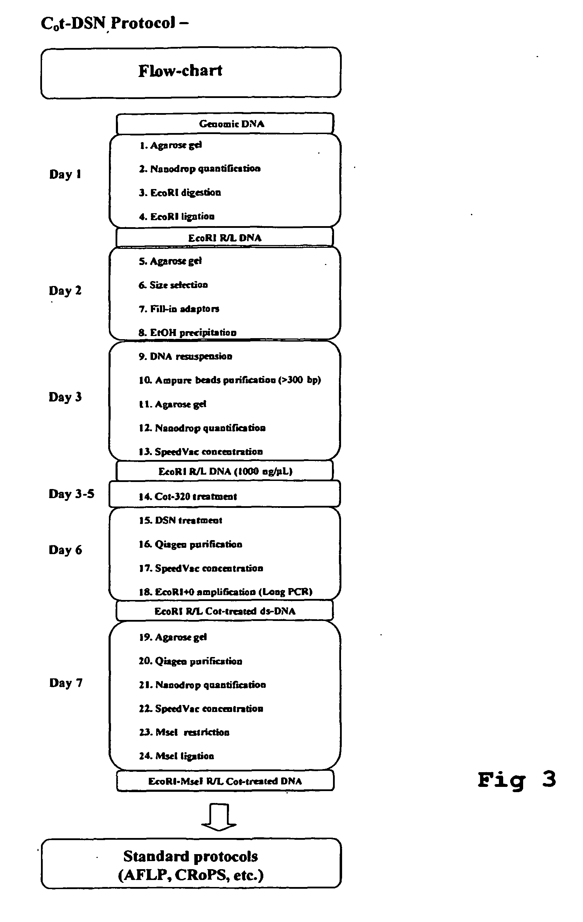

Figure 3 C₀t-DSN Protocol

### Examples

The invention will now be explained by the following examples. It will be clear to the skilled man that variations on this protocol are possible without departing from the gist of the invention,
1. Agarose gel
For DNA quality/integrity check, load 5µl DNA (RNAse treated!) on a 1% agarose gel.
2. Nanodrop quantification
For accurate quantification of DNA, dry and resuspend/ Speedvacuum concentrate the DNA to 1000 ng/µL if necessary (minimum concentration of 114 ng/µL)
3. EcoRI DNA digestion (60 µg DNA)

| Reagent | Conc. | Sample |
|---|---|---|
| 60 µg DNA | 1000 ng/µL | 60 µL |
| EcoRl | 50 U/µL | 6 µL |
| One-Phor-All | 10x | 60 µL |
| BSA | 10 mg/µL | 3 µL |
| DTT | 1 M | 3 µL |
| Milli Q water | | 468 µL |
| Total volume | | 600 µL |

Treat samples at 37°C for 6 hours.
- Use 5U of EcoRI per µg DNA [standard AFLP protocol requires 25U/µg DNA]
- Amount of DNA can vary from a minimum of 20 µg to more than 100 µg (scalable)
- Size-selection step between restriction and ligation is not performed

4. EcoRI adapter ligation

| Reagent | Conc. | Sample |
|---|---|---|
| EcoRI Adaptor | 5 µM | 300 |
| One-Phor-All | 10x | 100 |
| BSA | 10 mg/µL | 2 |
| DTT | 1 M | 2 |
| ATP | 10 mM | 300 |
| T4 ligase | 1 U/µL | 300 |
| Total volume | | 1004 |

The 1004 µL are added to the restricted 600 µL and treated at 37°C overnight.
- Use 40 ng DNA/pmole adaptor [like in the standard AFLP protocol]

5. Agarose gel
Check quality of the EcoRI digestion. Expect a smear between 1-10 kb with highest intensity at 2-3 kb.
6. Size-selection
Treat samples at 80°C for 20 min. in a water-bath.
7. Fill-in adaptors (ds adaptors synthesis)
In order to make ds-adaptor the following 300 µL solution is added to each tube containing 1.604mL R/L

| Reagent | Conc. | Sample |
|---|---|---|
| 10xPCR buffer ROCHE | | 190 |
| dNTPs | 5 mM | 70 |
| Taq Polymerase ROCHE | 5 U/µL | 7 |
| Milli Q water | | 33 |
| Total volume (1 x PCR) | | 300 |

Treat samples (1.904 mL) at 72°C for 30 min. in a water-bath, then chill tubes on ice and EtOH precipitate.
8. DNA EtOH precipitation of ds-RL samples
Transfer the 1.904 mL R/L in a 50 mL tube (blue cap, V bottom).
Add the same volume (1.9 mL) of 7.5 M Ammonium Acetate and mix. Add three volumes (11.4 mL) of 100% ethanol and let DNA precipitate at -20°C overnight. Centrifuge samples at 4800 rpm (large rotor) for 20 min. at 4°C.
Wash pellet with 1.0 mL cold 70% ethanol and carefully (!) transfer the pellet into a 1.5 mL eppendorf tube.
Centrifuge tubes at 14000 rpm (micro centrifuge) for 20 minutes at 4°C.
Carefully discard supernatant and let EtOH evaporate from pellets at 37°C for 15-20 minutes.
9. DNA resuspension
Re-suspend the pellet in 100 µL Milli Q water or EB buffer (QIAGEN) leaving tubes at RT for 3 hours or at 60°C for 20 min. and mixing slowly by pipetting.
10. Ampure Bead Purification of fragments > 300 bp (See Ampure Protocol)
At the 100 µL of the resuspended samples add 400 µL EB buffer (QIAGEN) and mix.
Add 350µL of Ampure beads and vortex (Ratio Beads/Sample = 0.7 to have cutoff ∼ 300 bp). Incubate samples for 5 min at RT.
Insert tubes in the (Magnetic Particle Concentrator) MPC for 5 min. to precipitate the beads. Remove supernatant by pipetting (leave tubes in the MPC).
Keep tubes in the MPC and wash beads with 1 mL 70% EtOH and discard supernatant (twice).
Dry pellets at 37°C for 10-15 minutes..
Remove tubes from the MPC and elute DNA with 200 µL Milli Q water (twice) by adding Milli Q water, vortexing/pipetting to resuspend all beads and leaving tubes in MPC for 5 min for beads precipitation.
Keep the supernatant (with DNA!) and place it in a new 1.5 mL tube (400 µL/sample).
11. Agarose gel
Check quality of purification (load 2µL on a 1% agarose gel).
12. Nanodrop quantification
For accurate DNA quantification.
13. SpeedVac concentration to 1000 ng/µL
Samples are concentrated to obtain ds-R/L purified DNA at a concentration of 1000 ng/µL. (Alternatively dry the sample and re-suspend it in the correct volume of Milli Q water).
14. Cₒt treatment
Cₒt treatment is performed at the following conditions:
1. 500 ng/µL DNA
2. 0.3 M NaCl
3. renaturation at 630C
At these conditions C₀t-320 takes 60 h 16m
Hybridization is performed as follow (in 200µL tubes) :
Sample
   DNA (at 1000 ng/µL) 2 µL
   2x C₀t Hybridization Buffer (0.6 M NaCl)
   Note: composition - 30% of 4x trimmer hybridization buffer at 2 M NaCl
   - 70% MQW 2 µL
   Total volume 4 µL
   Cover the 4 µL sample with 20 µL mineral oil and centrifuge (make sure the 4 µL are covered by oil).
   Treat samples at 95°C for 10 min (PCR thermocycler) and let hybridize at 63°C for the time required for the C₀t treatment (C₀t-320 = 60h 16m).

15. DSN treatment
Preheat the DSN Master Buffer at 63°C for >5 min.
Cₒt treatment is stopped (after 60h 16m 00s) placing the sample on ice for 30 seconds. Place samples at 63°C for 1 min.
Add 5 µL of preheated DSN Master Buffer to the 4 µL of renaturating solutions, mix quickly by pipetting
and incubate at 63°C for additional 10 min.
Add 1 µL DSN Enzyme, mix quickly by pipetting and centrifuge (make sure all the 10 µL are on the bottom of the tube) and treat at 68°C for 25 min.
Add 10µL of Stop Solution, mix and centrifuge and keep at 68°C for additional 5 min before placing tubes on ice.
Add 20 µL sterile water [total volume after DSN treatment = 40 µL] and purify the sample.
16. Qiagen purification
Follow Qiagen purification instruction and elute in 150 µL Milli Q water (three 50 µL elutions).
17. SpeedVac concentration
Concentrate the 150µL to 31 µL (-40 min. at 35°C). Adjust volume to 31 µL if necessary.
18. EcoRI+0 ds-synthesis of the DSN-treated ss-DNA samples using
Advantage 2 PCR kit
30 µL (of the 31 µL eluted from Qiagen purification) is +0 amplified to make ds-DNA as follow.
The remaining 1 µL (Not +0 amplified) will be used for agarose analysis
Sample

| | | |
|---|---|---|
| Purified ss DNA from DSN treatment | | 30 |
| EcoRI+0 E00L | (50 ng/µL) | 1.5 |
| 10x Ad2 buffer | | 5 |
| 50x dNTP | | 1 |
| 50x Ad2 Polymerase mix | | 1 |
| MQW | | 11.5 |
| total | | 50 |

The samples are treated at 94°C for 30 sec. then 24 cycles at 94°C for 7 sec., 56°C for 30 sec. and 72°C for 10 min., with last step at 72°C for 10 min and stored at 4°C.
19. Agarose gel
Load 1µL of "Not +0 amplified" and 1.6 µL "+0 amplified" for each sample.
20. Qiagen purification
Follow Qiagen purification instruction and elute in 150 µL Milli Q water (three 50 µL elutions).
21. Speedvacuum concentration
Concentrate the 150µL to 40-50 µL (minimum DNA concentration of 14 ng/µL).
22. Nanodrop quantification
For accurate DNA quantification.
23. Msel Restriction (Standard AFLP protocol)

| Reagent | Conc. | Sample. |
|---|---|---|
| C₀t treated DNA (500 ng) + Milli Q water | | 35.6 |
| Msel | 40 U/µL | 0.03 |
| One-Phor-All | 10x | 4 |
| BSA (µL) | 10 mg/µL | 0.2 |
| DTT (µL) | 1 M | 0.2 |
| Total volume | | 40 |

Samples are treated at 37°C for 1.0 h
Size-selection step between restriction and ligation is not performed
24. Msel adapter Ligation (Standard AFLP protocol)

| Reagent | Conc. | Sample |
|---|---|---|
| Msel Adapter | 50 µM | 1 |
| One-Phor-All | 10x | 1 |
| BSA | 10 mg/µL | 0.05 |
| DTT | 1 M | 0.05 |
| ATP | 10 mM | 1 |
| T4 ligase | 1 U/µL | 1 |
| MilliQ Water | | 5.9 |
| Total volume | | 10 |

The 10 µL are added to the restricted 40 µL and treated at 37°C overnight.
Starting from the C₀t-treated R/L samples, the standard protocols (i.e. for AFLP, CRoPS, etc. can be followed)
The reduction of repetitive sequences was performed by selecting samples from pepper and maize (both highly repeated genomes). The composition of convention R/L mixes (restriction-ligation mixtures) prior to any Cₒt treatment was

| | |
|---|---|
| EcoRl/Msel | 100-1000 bp |
| Pstl/Msel | 100-1000 bp |
| Msel-only with partial digestion | 1000 bp average |
| EcoRl-only | 2000-3000 bp average |
| Pstl-only | 4000-5000 bp average |

Cₒt parameter ranging from 0-320 (0, 1, 2, 5, 10, 20, 40, 80, 160, 320)

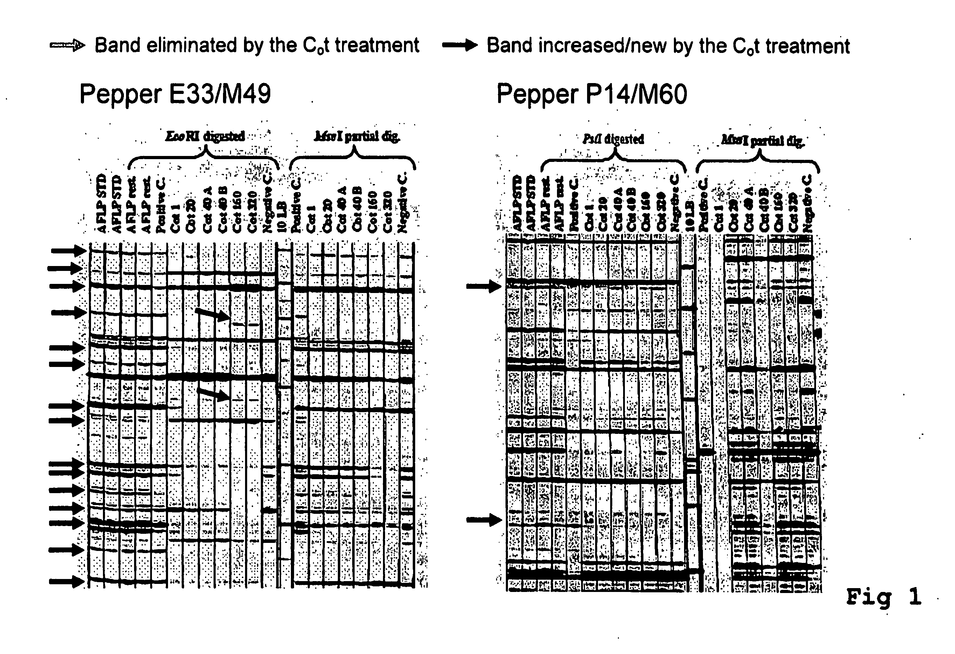

Pepper results for Pepper E33/M49 and Pepper P14/M60 are displayed in Fig 1.

The various restriction enzyme digests and Cₒt denaturation times were tested on two pepper samples that were restricted with EcoRI (pepper E33/M49) and amplified using primer E33 (E-AAG) and M49 (M-CAG) and with Pstl (pepper P14/M60) and amplified using primer P14 (P-AT) and M60 (M-CTC). From Fig 1 it is clear that repetitive forms are reduced and some hitherto undiscovered low-copy bands appear

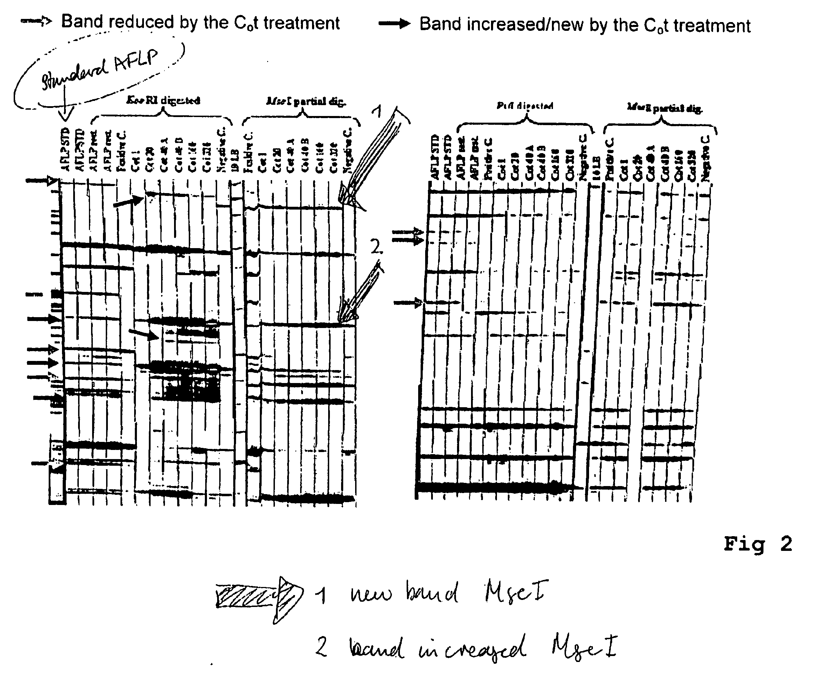

Maize results for Maize E35/M48 and Maize P12IM50 are displayed in Fig 2.

The various restriction enzyme digests and Cₒt denaturation times were tested on two Maize samples that were restricted with EcoRI (Maize E35/M48) and amplified using primer E35 (E-ACA) and M48 (M-CAC) and with PstI (Maize P12/M50) and amplified using primer P12 (P-AC) and M50 (M-CAT). From Fig 2 it is clear that repetitive forms are reduced and some hitherto undiscovered low-copy bands appear

From these results it was concluded that

| Pre-treatment | Cₒt effect |
|---|---|
| EcoRl/Msel digestion | No |
| Pstl/Msel digestion | No |
| Msel-partial | No |
| EcoRl only | Yes |
| Pst-only | Yes |
| Cₒt time | 320 |

| | No. Bands AFLP std. | No. bandsat Cₒt 320 | New bandsat Cₒt 320 | % Reduction of bands by Cₒt treatment | No. variable bands by C₀t treatment(variable intensity) |
|---|---|---|---|---|---|
| Pepper E35/M48 | 82 | 34 | 0 (0%) | 58% | 9 (27%) |
| Pepper E33/M49 | 81 | 49 | 3 (4%) | 43% | 16 (33%) |
| Maize E35/M48 | 39 | 30 | (8%) | 31% | 9 (30%) |
| Maize E33/M49 | 30 | 18 | 2 (7%) | 47% | 3 (17%) |
| Cₒt effect | | | ∼5% | 40 to 50% | 20 to 30% |

These results indicate that the Cₒt treatment determined the disappearance of 40-50 % of the standard AFLP bands and the appearance of 5% on new bands. A total of 20-30 % of the bands that remained after the Cₒt treatment presented a variation (increased or decreased) of band intensity.

Validation on qRT-PCR Pepper

| | STS single copy gene | Ribosomal repetitive | chloroplast repetitive |
|---|---|---|---|
| gDNA | 1 | 47.8 | 45.1 |
| R/L before treatment (EcoRl-only) | 1 | 49.8 | 43.8 |
| | | | |
| Normalized gDNA - No Cₒt | 1 | 60.5 | 67.4 |
| Normalized gDNA - Cₒt 320 | 1 | 13.6 | 23.7 |
| Cot effect | - 4.5 fold | - 4.5 fold | - 2.9 fold |
| R/L post treatment - No Cₒt | 1 | 13.5 | 34.1 |
| R/L post treatment - Cₒt 320 | 1 | 5.4 | 5 |
| Cot effect | | - 2.5 fold | - 6.8 fold |
| +1/+2 - No Cₒt | 1 | 67.0 | -115.9 |
| +1/+2 - Cₒt 320 | 1 | 16.3 | 41.8 |
| *Cₒt* effect | | - 41 fold | - 2.8 fold |

From these results is concluded that sequences that are ∼ 40 fold more abundant than single copy genes are reduced - 4 fold by the Cₒt treatment (40:1 to 10:1)

Further validation is achieved by Sanger sequencing.

## Claims

1. Method for the reduction of repetitive sequences (enhancing the (relative) amount of low-copy sequences) in adapter-ligated restriction fragments comprising the steps of:
a. restricting a starting DNA in a sample with a first restriction endonuclease and ligating a first adapter to the restriction fragments to obtain adapter-ligated restriction fragments;
b. performing a renaturation kinetics based fractionation on the adapter-ligated restriction fragments obtained in step (a);
c. restricting the restriction fragments obtained from step (b) with a second restriction endonuclease and ligating a second adapter to the restriction fragments;
d. obtaining a low-copy enriched fraction of adapter-ligated restriction fragments from the starting DNA.

2. Method according to claim 1,wherein after step (b) the renatured fractions of step (b) are subjected to a duplex specific nuclease (DSN);.

3. Method according to claim 1 or 2, wherein the first restriction endonuclease is a rare cutter and/or the second restriction endonuclease is a frequent cutter.

4. Method according to claims 1-3, wherein the renaturation kinetics based fractionation is a Cₒt treatment.

5. Method according to claim 4 wherein the Cₒt value of the Cₒt treatment is 320.

6. Method according to claims 1-5, wherein the low-copy fraction of the adaptor-ligated restriction fragments obtained from starting DNA is amplified using primers directed to the adapters having optionally 1-10 selective nucleotides at the 3'end

7. Method according to any of the previous claims, wherein the starting DNA is genomic DNA, cDNA, BAC DNA, mitochondrial DNA, chloroplast DNA or mixtures of plant genomic DNA with mitochondrial DNA and chloroplast DNA

8. Method according to any of the previous claims, wherein DSN normalisation is performed using a treatment comprising a duplex specific nuclease.

9. Use of the method as defined in claims 1-8 to generate AFLP fingerprints enriched for low-copy sequences.

10. Use of the method as defined in claims 1-8 as a treatment of a DNA sample prior to marker conversion or SNP mining.

## Patentansprüche

1. Verfahren zur Reduktion von repetitiven Sequenzen (Erhöhen der (relativen) Menge von Sequenzen niedriger Kopienzahl) in Adapter-ligierten Restriktionsfragmenten, umfassend die Schritte
a. Restringieren einer Ausgangs-DNA in einer Probe mit einer ersten Restriktionsendonuclease und Ligieren eines ersten Adapters an die Restriktionsfragmente, um Adapter-ligierte Restriktionsfragmente zu erhalten;
b. Durchführen einer auf Renaturierungskinetik beruhenden Fraktionierung mit den in Schritt (a) erhaltenen Adapter-ligierten Restriktionsfragmenten;
c. Restringieren der von Schritt (b) erhaltenen Restriktionsfragmente mit einer zweiten Restriktionsendonuclease und Ligieren eines zweiten Adapters an die Restriktionsfragmente;
d. Erhalten einer für niedrige Kopienzahl angereicherten Fraktion Adapter-ligierter Restriktionsfragmente von der Ausgangs-DNA.

2. Verfahren gemäß Anspruch 1, wobei die renaturierten Fraktionen von Schritt (b) nach Schritt (b) einer duplexspezifischen Nuclease (DSN) unterworfen werden.

3. Verfahren gemäß Anspruch 1 oder 2, wobei es sich bei der ersten Restriktionsendonuclease um eine selten schneidende und/oder bei der zweiten Restriktionsendonuclease um eine häufig schneidende handelt.

4. Verfahren gemäß den Ansprüchen 1 - 3, wobei es sich bei der auf Renaturierungskinetik beruhenden Fraktionierung um eine Cₒt-Behandlung handelt.

5. Verfahren gemäß Anspruch 4, wobei der Cₒt-Wert der Cₒt-Behandlung 320 beträgt.

6. Verfahren gemäß den Ansprüchen 1 - 5, wobei die von der Ausgangs-DNA erhaltene Fraktion Adapter-ligierter Restriktionsfragmente mit niedriger Kopienzahl unter Verwendung von gegen die Adapter gerichteten Primern, die gegebenenfalls 1 - 10 selektive Nucleotide am 3'-Ende aufweisen, amplifiziert wird.

7. Verfahren gemäß einem der vorhergehenden Ansprüche, wobei es sich bei der Ausgangs-DNA um genomische DNA, cDNA, BAC-DNA, mitochondriale DNA, Chloroplasten-DNA oder Gemische genomischer Pflanzen-DNA mit mitochondrialer und Chloroplasten-DNA handelt.

8. Verfahren gemäß einem der vorhergehenden Ansprüche, wobei die DSN-Normalisierung unter Verwendung einer Behandlung durchgeführt wird, die eine duplexspezifische Nuclease umfasst.

9. Verwendung des in den Ansprüchen 1 - 8 definierten Verfahrens, um AFLP-Fingerabdrücke zu erzeugen, die für Sequenzen mit niedriger Kopienzahl angereichert sind.

10. Verwendung des in den Ansprüchen 1 - 8 definierten Verfahrens als Behandlung einer DNA-Probe vor Markerkonversion oder SNP-Suchverfahren.

## Revendications

1. Procédé pour la réduction des séquences répétitives (augmentation de la quantité (relative) des séquences en faible nombre de copies) dans des fragments de restriction ligaturés à un adaptateur, comprenant les étapes:
a. restreindre un ADN de départ dans un échantillon avec une première endonucléase de restriction et ligaturer un premier adaptateur aux fragments de restriction pour obtenir des fragments de restriction ligaturés à un adaptateur ;
b. effectuer un fractionnement basé sur la cinétique de renaturation sur les fragments de restriction ligaturés à un adaptateur obtenus à l'étape (a) ;
c. restreindre les fragments de restriction obtenus à l'étape (b) avec une deuxième endonucléase de restriction et ligaturer un deuxième adaptateur aux fragments de restriction ;
d. obtenir une fraction enrichie en copies en faible nombre des fragments de restriction ligaturés à des adaptateurs issus de l'ADN de départ.

2. Procédé selon la revendication 1, dans lequel après l'étape (b) les fractions renaturées de l'étape (b) sont soumises à une nucléase spécifique des duplex (DSN).

3. Procédé selon la revendication 1 ou 2, dans lequel la première endonucléase de restriction est une enzyme de restriction de coupure rare et/ou la deuxième endonucléase de restriction est une enzyme de restriction de coupure fréquente.

4. Procédé selon les revendications 1 à 3, dans lequel le fractionnement basé sur la cinétique de renaturation est un traitement Cₒt.

5. Procédé selon la revendication 4, dans lequel la valeur de Cₒt du traitement Cₒt est 320.

6. Procédé selon les revendications 1 à 5, dans lequel la fraction à faible nombre de copies des fragments de restriction ligaturés à des adaptateurs obtenue à partir de l'ADN de départ est amplifiée en utilisant des amorces dirigées vers les adaptateurs ayant éventuellement 1 à 10 nucléotides sélectifs à l'extrémité 3'.

7. Procédé selon l'une quelconque des revendications précédentes, dans lequel l'ADN de départ est un ADN génomique, un ADNc, un ADN de BAC, un ADN mitochondrial, un ADN chloroplastique ou des mélanges d'ADN génomique végétal avec de l'ADN mitochondrial et l'ADN chloroplastique.

8. Procédé selon l'une quelconque des revendications précédentes, dans lequel la normalisation par DSN est effectuée en utilisant un traitement comprenant une nucléase spécifique des duplex.

9. Utilisation du procédé selon les revendications 1 à 8 pour générer des empreintes AFLP enrichies en séquences en faible nombre de copies.

10. Utilisation du procédé selon les revendications 1 à 8 en tant que traitement d'un échantillon d'ADN avant la conversion des marqueurs ou l'extraction des SNP.
